# EUROPÄISCHE PATENTSCHRIFT

(11) **EP 2 681 587 B1**
(45) Veröffentlichungstag und Bekanntmachung des Hinweises auf die Patenterteilung: **27.02.2019**
(21) Anmeldenummer: 12712061.6
(22) Anmeldetag: 02.03.2012
(51) Int. Cl.: G01T 1/24

(54) **STRAHLUNGSDETEKTOR UND MESSEINRICHTUNG ZUR DETEKTION VON RÖNTGENSTRAHLUNG**
RADIATION DETECTOR AND MEASUREMENT DEVICE FOR DETECTING X-RAY RADIATION
DÉTECTEUR DE RAYONNEMENT ET DISPOSITIF DE MESURE POUR LA MESURE DE RAYONS X

(30) Priorität: 04.03.2011 DE 102011013057
(43) Veröffentlichungstag der Anmeldung: 08.01.2014
(73) Patentinhaber: Thalhammer, Stefan, 80639 München (DE)
(72) Erfinder: THALHAMMER, Stefan, 80639 München (DE); HOFSTETTER, Markus, 84432 Hohenpolding (DE); HOWGATE, John, 80335 München (DE); STUTZMANN, Martin, 85435 Erding (DE)
(74) Vertreter: Hertz, Oliver
(86) Internationale Anmeldenummer: PCT/EP2012/000945
(87) Internationale Veröffentlichungsnummer: WO 2012/119740

(56) Entgegenhaltungen:
- EP-A1- 1 376 155
- WO-A2-2010/142773
- FR-A1- 2 911 965
- FR-A1- 2 945 128
- JP-A- 2010 067 738
- US-A1- 2008 157 253
- HOFSTETTER MARKUS ET AL: "Real-time x-ray response of biocompatible solution gate AlGaN/GaN high electron mobility transistor devices", APPLIED PHYSICS LETTERS, AIP, AMERICAN INSTITUTE OF PHYSICS, MELVILLE, NY, US, Bd. 96, Nr. 9, 5. März 2010 (2010-03-05), Seiten 92110-92110, XP012132404, ISSN: 0003-6951, DOI: 10.1063/1.3334682 in der Anmeldung erwähnt

## Beschreibung

Die Erfindung betrifft einen Strahlungsdetektor zur Detektion von Röntgenstrahlung, der mit einem GaN-basierten Halbleitermaterial aufgebaut ist. Des weiteren betrifft die Erfindung eine Messeinrichtung, die mindestens einen derartigen Strahlungsdetektor enthält, und Anwendungen des Strahlungsdetektors und der Messeinrichtung.

Es ist bekannt, Galliumnitrid (GaN) in Halbleiterdetektoren zur Detektion von Röntgenstrahlung zu verwenden. Beispielsweise wird in US 2010/0069749 A1 ein GaN-Sensor beschrieben, der in Reaktion auf eine Röntgenbestrahlung Lumineszenzlicht emittiert. Das Lumineszenzlicht wird vom Sensor über einen Lichtleiter zu einem Photodetektor geleitet. Diese Technik hat Nachteile, da die Kombination des Sensors dem Lichtleiter einen empfindlichen Aufbau darstellt und da die detektierte Röntgenstrahlung nicht direkt in ein elektrisches Messsignal umgewandelt wird.

Des weiteren wird von J.-Y. Duboz et al. in "Applied Physics Letters" Band 92, 2008, S. 263501, die Eignung von GaN zur Detektion von Röntgenstrahlung untersucht. Hierzu wurden GaN-Schichten, z. B. mit einer Dicke von 110 µm oder 480 µm, auf Silizium- oder Saphir-Substraten abgeschieden und mit Kontaktelektroden versehen, die mit der GaN-Schicht einen Schottky-Kontakt bildeten. Es wurde jedoch festgestellt, dass eine zuverlässige Detektion auf Röntgenstrahlung mit einer Energie unterhalb von 20 keV beschränkt war. Für praktische Anwendungen eines Strahlungsdetektors, zum Beispiel in der Dosimetrie, ist jedoch eine Empfindlichkeit für Röntgenstrahlung mit einer Energie oberhalb von 20 keV erforderlich.
Schließlich wird von M. Hofstetter et al. in "Applied Physics Letters" Band 96, 2010, S. 092110, ein Strahlungsdetektor für Röntgenstrahlung beschrieben, der einen sogenannten HEMT ("High Electron Mobility Transistor") mit einem GaN-basierten Mehrschichtsystem enthält. Dieser Strahlungsdetektor kann ebenfalls aufgrund seines Mehrschichtaufbaus Nachteile haben.
In der Praxis hat sich gezeigt, dass die bisher beschriebenen Strahlungsdetektoren auf GaN-Basis insbesondere aufgrund ihres komplexen Aufbaus, einer komplizierten Kalibrierung und/oder einer unzureichenden Empfindlichkeit für einen routinemäßigen Einsatz in der Dosimetrie ungeeignet sind. Des weiteren sind bisher keine praktikablen Anwendungen von GaN-basierten Strahlungsdetektoren, zum Beispiel in der Medizintechnik, der Materialprüfung oder der Strahlungsüberwachung beschrieben worden.
Weitere GaN-basierte Strahlungsdetektoren sind aus WO 2010/142773 A2 und US 2008/0157253 A1 bekannt. Die herkömmlichen Strahlungsdetektoren sind zur Verwendung in Messgeräten, wie zum Beispiel einer Kamera, oder einer Zellkultur ausgelegt. Ein anderer Strahlungsdetektor für die Dosimetrie ist aus FR 2 945 128 bekannt.

Die Aufgabe der Erfindung ist es, einen verbesserten Strahlungsdetektor anzugeben, mit dem Nachteile herkömmlicher Strahlungsdetektoren überwunden werden. Der Strahlungsdetektor soll sich insbesondere durch einen vereinfachten Aufbau, einen vereinfachten Betrieb und/oder eine erhöhte Empfindlichkeit im Vergleich zu herkömmlichen Detektoren auszeichnen. Die Aufgabe der Erfindung ist es des Weiteren, Messeinrichtungen und Anwendungen des Strahlungsdetektors anzugeben, mit denen Nachteile herkömmlicher Techniken zur Detektion von Röntgenstrahlung überwunden werden.

Diese Aufgaben werden durch einen Strahlungsdetektor mit den Merkmalen von Anspruch 1 beziehungsweise durch eine Messeinrichtung, die mindestens einen derartigen Strahlungsdetektor enthält, gelöst. Vorteilhafte Ausführungsformen und Anwendungen der Erfindung ergeben sich aus den abhängigen Ansprüchen. Gemäß einem ersten allgemeinen Gesichtspunkt der Erfindung wird ein Strahlungsdetektor zur Detektion von Röntgenstrahlung, bereitgestellt, der ein Trägersubstrat, eine auf dem Trägersubstrat angeordnete GaN-basierte Detektorschicht und mit der Detektorschicht verbundene Kontaktelektroden umfasst. Gemäß der Erfindung hat die Detektorschicht eine Dicke, die geringer als 50 µm ist. Des weiteren ist gemäß der Erfindung vorgesehen, dass die Kontaktelektroden mit der Detektorschicht ohmsche Kontakte bilden. Die Erfinder haben festgestellt, dass eine einzige, mit ohmschen Kontakten versehene Detektorschicht mit der genannten, im Vergleich zu herkömmlichen Strahlungsdetektoren erheblich verringerten Schichtdicke eine empfindliche, reproduzierbare und nur eine einfache Widerstands- oder Leitfähigkeitsmessung erfordernde Detektion von Röntgenstrahlung ermöglicht. Bei Anlegen einer Spannung an die mit den Kontakten versehene Detektorschicht liefert eine Strommessung einen Widerstands- oder Leitfähigkeitswert. Die Detektorschicht wirkt wie ein Photoleiter, in dem in Reaktion auf Röntgenbestrahlung ein Photostrom generiert wird, der an den Kontaktelektroden messbar ist. Die detektierte Röntgenstrahlung wird somit direkt in ein elektrisches Messsignal (Detektorstrom, Widerstands- oder Leitfähigkeitswert) konvertiert. Die Konvertierungsausbeute ist dabei so groß, dass der erfindungsgemäße Strahlungsdetektor miniaturisierbar ist und für zahlreiche Anwendungen in der Dosimetrie angepasst werden kann. Vorzugsweise ist der Strahlungsdetektor für eine Detektion von Röntgenstrahlung im Energiebereich von 1 keV bis 300 keV, insbesondere oberhalb von 50 keV ausgelegt.

Vorteilhafterweise stellt der erfindungsgemäße Strahlungsdetektor einen Strahlensensor dar, der die photoleitenden Eigenschaften von "wide bandgap" Halbleitern nutzt. Unter Strahleneinfluss ändert sich das leitende Detektorvolumen. Physikalisch ist dabei keine fest definierte elektrische Sperrschicht wie bei herkömmlichen Halbleiterdetektoren notwendig. Dies ermöglicht einen neuartigen Detektionsmodus für die Dosimetrie. Die Detektion der Strahlung beruht auf dem Prinzip eines Photoleiters mit internen Verstärkungseigenschaften. Es ist keine elektrische Sperrschicht erforderlich, sondern eine Volumen-unabhängige Messung über die ohmschen Kontakte vorgesehen. Das Messsignal kann in verschiedener Weise (z. B. elektronisch, graphisch, akustisch) dargestellt werden.

Das Funktionsprinzip des erfindungsgemäßen GaN-Strahlungsdetektors unterscheidet sich fundamental von dem der konventionell erhältlichen Halbleiterdetektoren für Röntgenstrahlung, bei denen Photo-induzierte Ladungsträger über ein elektrisches Feld gesammelt werden. Dagegen findet bei den GaN-Sensoren eine durch Strahlung hervorgerufene Änderung des Widerstandes statt (Photokonduktor), indem das Detektorvolumen verändert wird, in dem der Ladungstransport stattfindet. Obwohl auch in den GaN-Sensoren Raumladungszonen auftreten (in erster Linie durch Oberflächeneffekte), passiert der elektrische Strom parallel zu diesen Raumladungszonen den Halbleiter. Die Bestrahlung führt zu einem Nicht-Gleichgewicht von freien Landungsträgerkonzentrationen, die das Gesamtvolumen der Raumladungszonen und somit das Volumen verändern, welches zum Ladungstransport beiträgt. Daraus ergibt sich, dass die Höhe des Messsignals nicht direkt auf die Erzeugung von freien Ladungsträgern beschränkt ist, sondern eine massive interne Verstärkung stattfinden kann, wodurch im Vergleich zu herkömmlichen Techniken erhöhte Detektionssensitivitäten möglich sind.

Gemäß einem zweiten allgemeinen Gesichtspunkt der Erfindung wird eine Messeinrichtung bereitgestellt, die mit mindestens einem Strahlungsdetektor gemäß dem ersten Gesichtspunkt der Erfindung ausgestattet ist. Die Messeinrichtung ist allgemein eine Untersuchungseinrichtung, die zur Dosimetrie und optional für weitere Funktionen, wie z. B. eine Kultivierung biologischer Zellen, eingerichtet ist. Aufgrund der Miniaturisierbarkeit des erfindungsgemäßen Strahlungsdetektors ist die Messeinrichtung insbesondere zur ortsaufgelösten Dosimetrie geeignet.

Gemäß einer bevorzugten Ausführungsform der Erfindung ist die Dicke der Detektorschicht geringer als 10 µm, insbesondere geringer als 5 µm. Vorzugsweise ist die Dicke der Detektorschichten größer als 100 nm, insbesondere größer als 500 nm. Die geringe Dicke der GaN-basierten Detektorschicht bietet Vorteile sowohl in Bezug auf die Herstellung des Strahlungsdetektors als auch in Bezug auf dessen Integration in der Messeinrichtung. Gemäß weiteren bevorzugten Ausführungsformen der Erfindung hat die Detektorschicht eine Fläche, die geringer als 100 mm², insbesondere geringer als 10 mm² ist. Die Fläche ist vorzugsweise größer als 1 µm², insbesondere größer als 0.1 mm².

Vorzugsweise umfasst der Strahlungsdetektor eine einzige Detektorschicht. Mit anderen Worten, es ist ausschließlich eine Schicht vorgesehen, die mit ohmschen Kontakten ausgestattet und zur Erzeugung des Messsignals vorgesehen ist. Besonders bevorzugt besteht die Detektorschicht aus GaN, das optional eine Dotierung, zum Beispiel aus Eisen oder Kohlstoff, enthalten kann.

Weitere Vorteile für die Miniaturisierung des Strahlungsdetektors ergeben sich, wenn die Kontaktelektroden aus zwei Kontaktelektroden bestehen, die einseitig, das heißt insbesondere auf der zum Trägersubstrat entgegengesetzten Seite der Detektorschicht angeordnet sind. Die Kontakte befinden sich gemeinsam auf der selben Oberfläche der Detektorschicht.

Vorteilhafterweise kann der Strahlungsdetektor mit mindestens einem der folgenden Merkmale ausgestattet sein, um den Strahlungsdetektor für eine bestimmte Anwendung gezielt anzupassen. Gemäß einer ersten Variante kann der Strahlungsdetektor eine Verkapselung aufweisen. Die Verkapselung umfasst eine Hülle, zum Beispiel aus Kunststoff, welche das Trägersubstrat und die Detektorschicht teilweise oder allseitig einschließt. Der Strahlungsdetektor mit der Verkapselung bildet bei drahtloser Signalübertragung ein autarkes Bauteil. Alternativ ragen bei einem leitungsgebundenen Betrieb lediglich Verbindungsleitungen zum Anschluss der Kontaktelektroden in die Umgebung der Verkapselung. Vorteilhafterweise kann der Strahlungsdetektor mit der Verkapselung mit einer Dicke unterhalb von 10 mm, insbesondere 1 mm gebildet sein, was besondere Vorteile für die medizinische Dosimetrie bietet. Besonders bevorzugt ist die Verkapselung flüssigkeitsdicht, resistent gegen Säuren, resistent gegen Basen, temperaturbeständig und/oder druckbeständig. Dies ermöglicht vorteilhafterweise die Anwendung des Strahlungsdetektors in extremen Umgebungsbedingungen, zum Beispiel zur Dosimetrie in einem chemischen Reaktor.

Gemäß der Erfindung ist der Strahlungsdetektor mit mindestens einer Haftoberfläche ausgestattet. Die Bereitstellung der Haftoberfläche bedeutet, dass der Strahlungsdetektor und/oder optional die Verkapselung des Strahlungsdetektors auf mindestens einer Oberfläche mit einer inhärenten Haftfähigkeit gebildet ist. Die Haftoberfläche weist zum Beispiel ein Klebemittel auf, das ein Anhaften des Strahlungsdetektors an einem Gegenstand, zum Beispiel auf der Oberfläche eines Untersuchungsgegenstands oder eines Probanden, erlaubt.

Des Weiteren ist gemäß der Erfindung der Strahlungsdetektor mit einer elektronischen Schaltung ausgestattet, die zum Beispiel mit dem Trägersubstrat verbunden oder optional in der Verkapselung eingeschlossen ist. Die elektronische Schaltung ist zur Strommessung, Datenspeicherung und Datenübertragung eingerichtet. Die Strommessung bedeutet, dass mit der elektronischen Schaltung ein elektrisches Messsignal erfassbar ist, das für den in Reaktion auf Röntgenstrahlung in der Detektorschicht erzeugten Photostrom charakteristisch ist. Die elektronische Schaltung ist gemäß der Erfindung zur drahtlosen Kommunikation mit einer externen Steuereinrichtung konfiguriert und umfasst hierzu eine RFID-Einrichtung. Damit ergeben sich Vorteile für die Anwendung des Strahlungsdetektors in komplex aufgebauten Untersuchungsgegenständen.

Gemäß einer weiteren Variante der Erfindung kann der Strahlungsdetektor für eine energieaufgelöste Messung der Röntgenstrahlung ausgelegt sein. Bei einer Röntgenstrahlung mit einer Dosisrate oberhalb von z. B. rd. 0,2 mGy/s arbeitet der Detektor nahezu energieunabhängig. Trifft monochromatische Röntgenstrahlung mit bekannter Dosisrate auf den Detektor, lässt sich so die Energie der Strahlung ermitteln.

Gemäß einer weiteren Variante der Erfindung kann der Strahlungsdetektor mit einer biegsamen Materialbahn ausgestattet sein. Das Trägersubstrat kann mit der Materialbahn verbunden sein, die zum Beispiel ein flächiges oder streifenförmiges, ein- oder mehrschichtiges Textilmaterial umfasst. Die Verwendung der Materialbahn vereinfacht die Befestigung des Strahlungsdetektors an einem Untersuchungsgegenstand. Besonders bevorzugt ist die Haftoberfläche an der biegsamen Materialbahn vorgesehen. In diesem Fall ist das Trägersubstrat mit der Detektorschicht auf einem Klebeband angeordnet, das nach Art eines Verbandpflasters auf einem Probanden oder entsprechend auf einem anderen Untersuchungsgegenstand fixierbar ist. Das Klebeband kann eine Vielzahl von Strahlungsdetektoren tragen, sodass ein Detektorarray geschaffen wird.

Vorteilhafterweise kann die Verkapselung des Strahlungsdetektors eine biokompatible Oberfläche aufweisen. Die Verkapselung kann aus einem biokompatiblen Material, zum Beispiel PDMS, PEN, PET, Kunststoff, oder Photolack, bestehen oder eine Beschichtung aus dem biokompatiblen Material tragen. Vorteilhafterweise wird damit eine Beeinträchtigung eines biologischen Untersuchungsgegenstands, insbesondere bei der Implantation des Strahlungsdetektors in einen Organismus, vermieden.

Gemäß bevorzugten Varianten der Erfindung ist der Strahlungsdetektor Teil einer Endoskopieeinrichtung, Teil eines Klebebandes, Teil einer Kultivierungseinrichtung für biologische Zellen und/oder Teil einer Implantateinrichtung, die zur Implantation in oder an einem lebenden Organismus konfiguriert ist. Aufgrund der Miniaturisierbarkeit des Strahlungsdetektors kann dieser problemlos in einer Endoskopieeinrichtung angeordnet werden, um mit dieser dosimetrische Messungen im Innern eines Organismus oder eines anderen Untersuchungsgegenstands durchzuführen. Die Integration des Strahlungsdetektors in eine Kultivierungseinrichtung für biologische Zellen, wie z. B. einen Zellinkubator oder ein fluidisches Mikrosystem, dass zur Manipulation biologischer Zellen eingerichtet ist, ermöglicht vorteilhafterweise, dass dosimetrische Messungen unter den konkreten Kultivierungsbedingungen in Echtzeit durchführbar sind.

Gemäß einer bevorzugten Ausführungsform der erfindungsgemäßen Messeinrichtung ist diese mit einem Gehäuse ausgestattet, in dem der mindestens eine Strahlungsdetektor beweglich ist. Dies vereinfacht vorteilhafterweise die Anwendung der Messeinrichtung als Materialprüfgerät.

Gemäß einer weiteren bevorzugten Variante der Erfindung ist die Messeinrichtung mit einer Vielzahl von Strahlungsdetektoren ausgestattet, die entlang einer vorbestimmten Bezugslinie oder Bezugsfläche angeordnet sind. Die Strahlungsdetektoren können zum Beispiel ein Linien-Array entlang einer geraden oder gekrümmten Linie oder ein Flächen-Array entlang einer ebenen oder gekrümmten Bezugsfläche bilden. Diese Varianten der Erfindung sind für die bildgebende Dosimetrie unter Verwendung des erfindungsgemäßen Strahlungsdetektors von besonderem Vorteil.

Weitere bevorzugte Merkmale der erfindungsgemäßen Messeinrichtung umfassen eine Alarmeinrichtung, eine Positionierungseinrichtung und/oder eine Messwertaufnehmereinrichtung. Die Alarmeinrichtung ist dazu konfiguriert, bei Überschreiten unerwünschter Strahlungsdosiswerte einen Alarm, zum Beispiel einen optischen oder akustischen Alarm zu erzeugen. Vorteilhafterweise kann die erfindungsgemäße Messeinrichtung in diesem Fall einen Strahlungsmelder darstellen, der nach Art eines herkömmlichen Rauchmelders betrieben wird. Die Positionierungseinrichtung kann zur Positionierung des mindestens einen Strahlungsdetektors relativ zu einem zu untersuchenden Gegenstand angepasst sein. Die Positionierungseinrichtung kann zum Beispiel im Gehäuse der Messeinrichtung vorgesehen sein. Dies ermöglicht vorteilhafterweise ortsaufgelöste dosimetrische Messungen am Untersuchungsgegenstand. Schließlich ermöglicht die Messwertaufnehmereinrichtung die Messung von Zeitreihen von Dosiswerten, zum Beispiel zur Überwachung eines Untersuchungsgegenstands, wie zum Beispiel eines Raumes oder eines Trägers des Strahlungsdetektors.

Zusammengefasst hat der erfindungsgemäße Strahlungsdetektor die folgenden Vorteile. Der Strahlungsdetektor ist durch lithographische Prozesse herstellbar und daher einfach miniaturisierbar. Die Detektorgröße (insbesondere Ausdehnung der empfindlichen Fläche) ist nahezu uneingeschränkt, insbesondere im Bereich von mehreren hundert µm bis zu 1 µm, bevorzugt in der Größe von 30 µm, skalierbar. Es ist ein Betrieb in Flüssigkeiten, insbesondere Säuren und Basen möglich. Es sind kombinierte Potentialmessungen mit HEMTs möglich, dadurch sind Multiparametermessungen von Ionenveränderungen in z. B. Zellmedien möglich. Der erfindungsgemäße Strahlungsdetektor erfordert keine zusätzliche in-situ Elektronik nötig und kann räumlich-unabhängig von einer Steuerelektronik betrieben werden. Des Weiteren ist eine Kühlung des Strahlungsdetektors nicht erforderlich. Der erfindungsgemäße Strahlungsdetektor hat eine sehr geringe Winkelabhängigkeit des Detektorsignals von der einfallenden Strahlung, so dass ein universeller Einbau möglich ist.

Des Weiteren hat der erfindungsgemäße Strahlungsdetektor einen sehr großen Energiemessbereich, einen sehr großer Dosisratenmessbereich und nahezu keine Energieabhängigkeit im diagnostischen Röntgenbereich. Durch die extrem breiten Detektionsbereiche der GaN-Sensoren (Energie und Dosisrate), der Möglichkeit zur Biokompatibilisierung und die Möglichkeit zur Miniaturisierung, bieten die GaN-Sensoren ein erhebliches Potential im Bereich der Medizin sowie in medizin-technischen Anwendungen. Schließlich ermöglicht der Strahlungsdetektor eine 3-dimensionale Dosimetrie mit der Möglichkeit der Bildgebung.

Bei der Anwendung in der Diagnostik bei MRT-PET Kombinationen ergibt sich insbesondere der folgende Vorteil. Ein Problem bei herkömmlichen PET-Scannern ist die fehlende Kompatibilität von hohen Magnetfeldern und Photomultipliern, welche bisher in Kombination mit Szintillatoren als Detektoren dienen. Der Vorteil der Erfindung liegt darin, dass ein Detektor bereitgestellt wird, welcher unempfindlich gegenüber hohen Feldern ist, so dass ein "one-stop shop" MRT-PET-Scanner gebaut werden kann. Die Erfindung ermöglicht ferner die Integration von Gammadetektoren (PET/SPECT-Detektoren) in MRT-Scanner für gleichzeitige, funktionelle und morphologische Informationsgewinnung, eine Verkürzung der Untersuchungszeit, eine "one-stop shop"-Untersuchung, und, weil der Detektor und die Messelektronik separat platziert werden können, eine Verringerung der Detektorgröße.

Weitere Einzelheiten und Vorteile der Erfindung werden im Folgendem unter Bezug auf die in den beigefügten Zeichnungen dargestellten bevorzugten Ausführungsformen der Erfindung erläutert. Es zeigen in:
- Figuren 1A und 1B:: eine schematische Schnittansicht und eine schematische Draufsicht auf eine erste Ausführungsform eines erfindungsgemäßen Strahlungsdetektors;
- Figuren 2 bis 5:: schematische Illustrationen weiterer Ausführungsformen des erfindungsgemäßen Strahlungsdetektors;
- Figur 6:: eine schematische Illustrationen der Positionierung des erfindungsgemäßen Strahlungsdetektors am Körper eines Probanden;
- Figuren 7 bis 9:: bevorzugte Ausführungsformen erfindungsgemäßer Messeinrichtungen, die mindestens einen Strahlendetektor enthalten; und
- Figuren 10 bis 12:: schematische Illustrationen weiterer bevorzugter Anwendungen der Messeinrichtung.

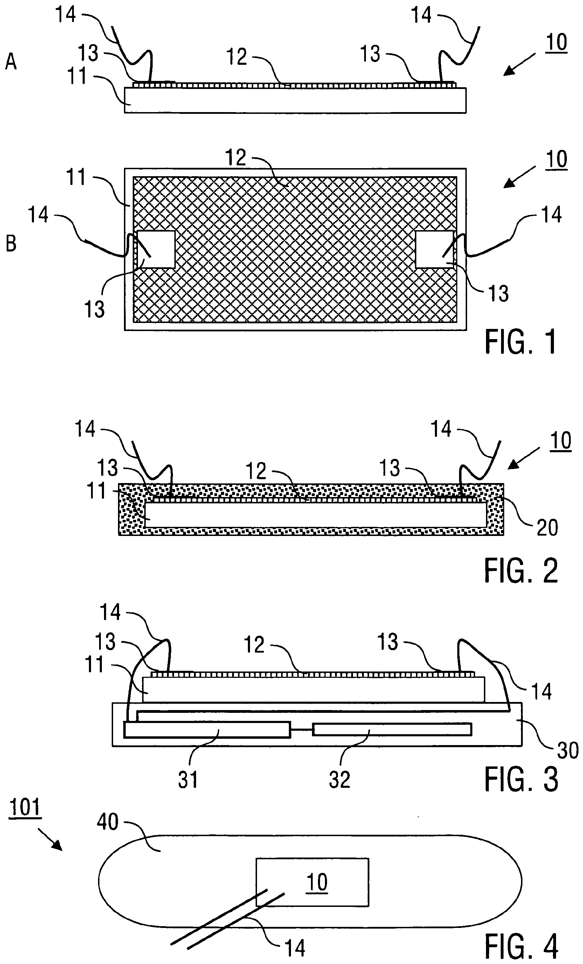

Gemäß den Figuren 1A und 1B umfasst der Strahlungsdetektor 10 ein Trägersubstrat 11, eine Detektorschicht 12 und Kontaktelektroden 13, die über Verbindungsleitungen 14 mit einer elektronischen Schaltung zur Strommessung (nicht dargestellt) verbunden werden können. Das Trägersubstrat 11 umfasst zum Beispiel Saphir mit einer Dicke von 0,33 mm. Die Detektorschicht 12 besteht aus GaN mit einer Dicke von zum Beispiel 2,5 µm. Die Kontaktelektroden 13 bestehen zum Beispiel aus Ti/Al. Sie sind mittels thermischem- oder Elektronenstrahl-Aufdampfen auf der GaN-Detektorschicht 12 aufgebracht, so dass zwischen den Kontaktelektroden 13 und der Detektorschicht 12 jeweils nach einem thermischen Ausheizen (Ausglühen) ein ohmscher Kontakt gebildet wird. Die Maße der Detektorschicht 12 (Figur 1B) betragen zum Beispiel 0,5 mm · 2 mm, während die Maße der Kontaktelektroden 13 zum Beispiel jeweils 300 µm · 300 µm betragen. Der Strahlungsdetektor 10 ist für Röntgenstrahlung mit einer Energie von 20 bis 300 keV bis in den µGy-Bereich empfindlich.

Figur 2 illustriert eine Ausführungsform der Erfindung, bei der der Strahlungsdetektor 10 mit einer Verkapselung 20 ausgestattet ist. Das Trägersubstrat 11 und die Detektorschicht 12 sowie die Kontaktelektroden 13 sind von der Verkapselung 20 vollständig eingeschlossen. Gemäß einer abgewandelten Variante der Erfindung kann eine partielle Verkapselung vorgesehen sein. Beispielsweise kann es bei Anwendungen des Strahlungsdetektors von Vorteil sein, wenn nur die elektrischen Kontakte verkapselt sind und die übrige sensitive Oberfläche zwischen den Kontakten unverkapselt ist. Die Verkapselung 20 besteht zum Beispiel aus Epoxidharz. Sie hat zum Beispiel eine Dicke von 0,5 mm. Wenn die Anwendung des Strahlungsdetektors 10 auf der Oberfläche oder im Inneren eines biologischen Organismus vorgesehen ist, trägt die Verkapselung 20 allseitig eine biokompatible Oberfläche, zum Beispiel aus PDMS.

Figur 3 illustriert eine Ausführungsform des Strahlungsdetektors 10, bei der das Trägersubstrat 11 mit einer elektronischen Schaltung 30 verbunden ist. Die Verbindungsleitungen 14 von den Kontaktelektroden 13 führen unmittelbar in die elektronische Schaltung 30, die beim dargestellten Beispiel mindestens einen Schaltkreis 31 und eine Antenne 32 umfasst. Der Schaltkreis 31 ist zur Messung des in Reaktion auf Röntgenstrahlung in der Detektorschicht 12 erzeugten Photostroms und zur Speicherung von Messwerten eingerichtet, die den Photostrom oder davon abgeleitete Größen repräsentieren. Der Schaltkreis 31 kann auch als Messwertaufnehmereinrichtung vorgesehen sein, mit der im Zeitverlauf wiederholt Dosiswerte aufgezeichnet werden (zeitaufgelöstes Monitoring). Die Antenne 32 ist für die drahtlose Kommunikation und mit einer externen Steuereinrichtung (nicht dargestellt) konfiguriert. Die elektronische Schaltung umfasst zum Beispiel einen RFID-Chip.

Figur 4 illustriert eine Messeinrichtung 101 mit einem Klebeband 40, auf dessen Oberfläche der schematisch gezeigte Strahlungsdetektor 10 angeordnet ist. Der Strahlungsdetektor 10 ist drahtlos oder wie dargestellt über Verbindungsleitungen 14 mit einer Steuereinrichtung (nicht dargestellt) verbunden, die zur Aufnahme von Messwerten vom Strahlungsdetektor 10 vorgesehen ist. Der Verbund aus dem Klebeband 40 mit dem Strahlungsdetektor 10 stellt eine auf der Oberfläche des Untersuchungsgegenstands, zum Beispiel eines Patienten, wie ein "Pflaster" fixierbare Messeinrichtung dar. Dies ermöglicht vorteilhafterweise eine auf der Oberfläche des Patienten ortsaufgelöst durchgeführte Dosimetrie, zum Beispiel während einer Bestrahlungsbehandlung.

Bei dieser Ausführungsform der Erfindung ist der Strahlungsdetektor 10 vorzugsweise für eine drahtlose Kommunikation mit der externen Steuereinrichtung ausgelegt. Das Klebeband 40 besteht vorzugsweise aus Gewebeband oder Textilband mit einer Klebeschicht, wie es von herkömmlichen Verbandmaterialien an sich bekannt ist.

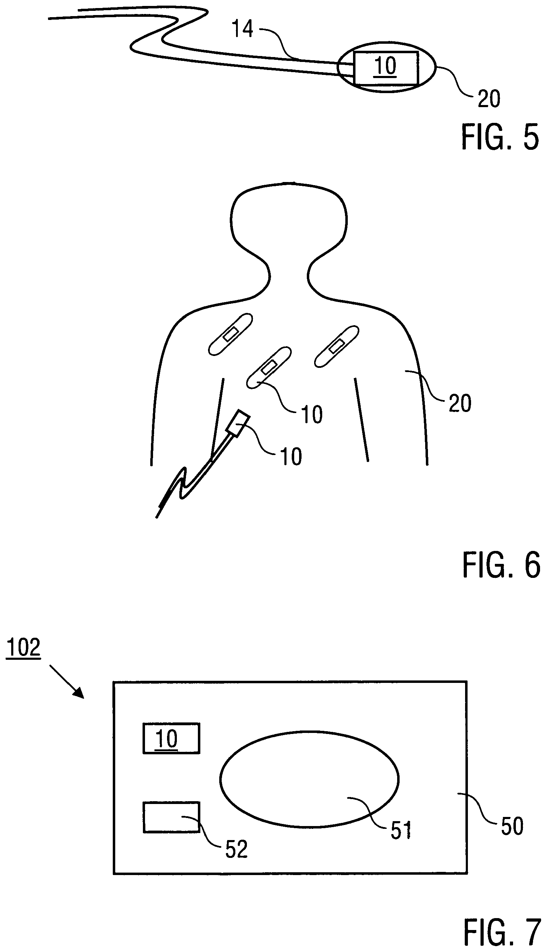

In Figur 5 ist der verkapselte Strahlungsdetektor 10 mit Verbindungsleitungen 14, die ggf. in einem einzelnen Kabel aus der Verkapselung 20 geführt sind, schematisch illustriert. Figur 6 veranschaulicht schematisch die Positionierung von Strahlungsdetektoren 10 gemäß den Figuren 4 oder 5 auf der Hautoberfläche es Probanden. Alternativ ist eine subkutane Anordnung oder eine Anordnung in einem Hohlraum des Probandenkörpers möglich.

Figur 7 illustriert den erfindungsgemäßen Strahlungsdetektor als Teil einer Endoskopieeinrichtung Teil einer Kultivierungseinrichtung 102 für biologische Zellen. Die Kultivierungseinrichtung 102 umfasst z. B. eine Platte 50, in oder auf der ein Kultivierungssubstrat 51, z. B. in Form einer Schale, der Strahlungsdetektor 10 und eine Sensoreinrichtung 52 zur Erfassung von Kultivierungsbedingungen angeordnet sind. Die Kultivierungseinrichtung 102 kann zum Beispiel als fluidisches Mikrosystem aufgebaut sein.

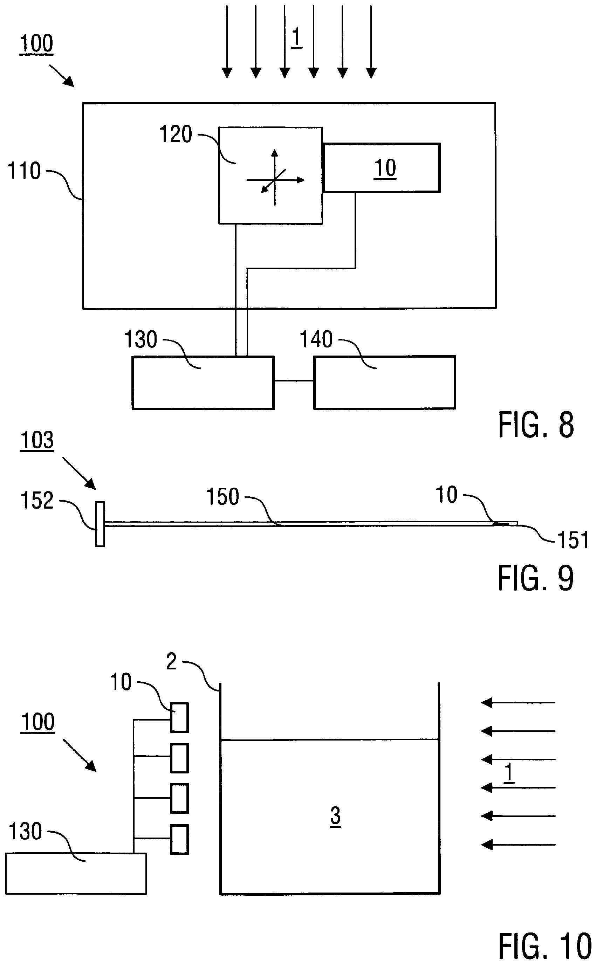

Figur 8 illustriert schematisch eine weitere Ausführungsform einer erfindungsgemäßen Messeinrichtung 100, die zum Beispiel für die Dosimetrie bei der Materialprüfung eingerichtet ist. Die Messeinrichtung 100 umfasst ein Gehäuse 110, in dem mindestens ein Strahlungsdetektor 10 beweglich angeordnet ist. Zur Positionierung des Strahlungsdetektors 10 im Gehäuse 110 ist eine Positionierungseinrichtung 120 vorgesehen, die zum Beispiel eine Stelleinrichtung zum Verfahren des Strahlungsdetektors 10 in allen drei Raumrichtungen umfasst. Die Positionierungseinrichtung 120 und der Strahlungsdetektor sind mit einer Steuereinrichtung 130 verbunden, die erstens der Steuerung der Positionierungseinrichtung 120 und zweitens der Aufnahme von Messwerten vom Strahlungsdetektor 10 dient. Die Steuereinrichtung 130 kann mit einer Ausgabeeinrichtung 140, wie zum Beispiel einer Anzeigeeinrichtung und/oder einem Drucker, verbunden sein. Die Steuereinrichtung 130 und die Ausgabeeinrichtung 140 können durch einen Computer, optional mit Zusatzeinrichtungen, realisiert werden.

Bei Positionierung der Messeinrichtung 100 im Strahlungsfeld von Röntgenstrahlung 1 kann der Strahlungsdetektor 10 im Strahlungsfeld verfahren werden, um zum Beispiel ein Strahlungsprofil aufzunehmen.

Für medizinische Anwendungen ist eine Ausführungsform der Messeinrichtung von besonderem Interesse, bei der diese eine Endoskopieeinrichtung 103 ist, wie schematisch in Figur 9 illustriert ist. Die Endoskopieeinrichtung 103 umfasst einen starren oder flexiblen Endoskopschaft 150 mit einem freien Ende 151, das zur Einführung in einen Untersuchungsgegenstand, zum Beispiel in das Innere eines Probanden vorgesehen ist, und mit einer am entgegengesetzten Ende vorgesehenen Kopplungseinrichtung 152 zur Verbindung der Endoskopieeinrichtung 102 mit einem medizinischen Untersuchungsgerät. Der Strahlungsdetektor 10 ist nahe am freien Ende 151 fixiert oder in Längsrichtung des Endoskopschafts 150 beweglich. Des Weiteren ist der Strahlungsdetektor 10 über Verbindungsleitungen, die durch den Endoskopschaft 150 verlaufen, mit einer Steuereinrichtung verbunden. Im letzteren Fall können im Untersuchungsgegenstand Strahlungsprofile aufgenommen werden, ohne das Endoskop zu bewegen. Die Endoskopieeinrichtung 102 wird verwendet, wie dies an sich von herkömmlichen Endoskopen bekannt ist, wobei erfindungsgemäß durch die Bereitstellung des Strahlungsdetektors 10 im Endoskopschaft eine neue Funktionalität der Endoskopieeinrichtung 103 erreicht wird.

Eine erfindungsgemäße Messeinrichtung 100 kann mit mehreren Strahlungsdetektoren 10 ausgestattet sein, wie schematisch in Figur 10 illustriert ist. Es sind zum Beispiel vier Strahlungsdetektoren 10 vorgesehen, die ein Detektorarray bilden und mit einer Steuereinrichtung 130 verbunden sind. Der Untersuchungsgegenstand stellt zum Beispiel ein Gefäß 2 dar, in dem sich eine Flüssigkeit 3 befindet. Bei Durchstrahlung des Gefäßes 2 mit Röntgenstrahlen 1 kann durch Auslesen der Strahlungsdetektoren 10 der Füllstand im Gefäß 2 ermittelt werden.

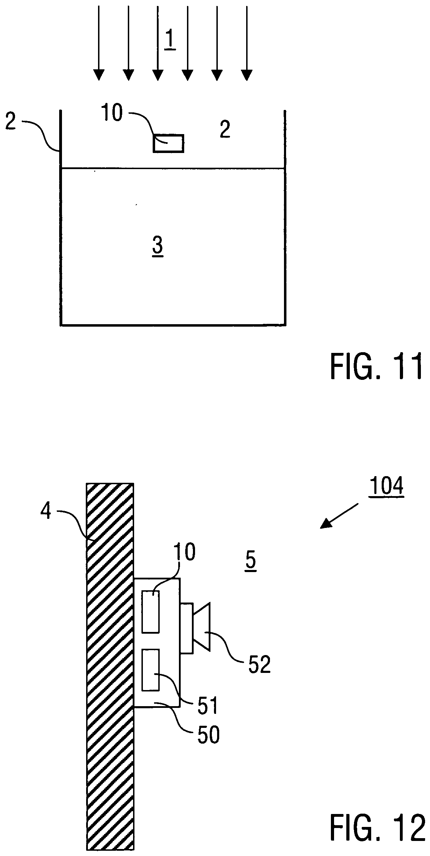

Eine weitere Anwendung des Strahlungsdetektors bei der Streustrahlungsmessung in der Nähe eines Untersuchungsgegenstands ist schematisch in Figur 11 illustriert. In diesem Fall ist der Untersuchungsgegenstand ebenfalls durch ein Gefäß 2 mit einer Flüssigkeit 3 illustriert, deren Oberfläche in Bezug auf die Rückstreuung von Röntgenstrahlung 1 untersucht werden soll. Der Strahlungsdetektor 10 kann über der Oberfläche des Untersuchungsgegenstands, insbesondere über der Oberfläche der Flüssigkeit 3 im Gefäß 2 bewegt werden, wobei aufgrund des geringen Eigenvolumens eine hohe Ortsauflösung erzielt werden kann.
Schließlich illustriert Figur 12 die Anwendung der erfindungsgemäßen Messeinrichtung als Strahlenmelder 104. Der Strahlungsdetektor 10 ist gemeinsam mit einer elektronischen Schaltung 61 in einem gemeinsamen Gehäuse 60 angeordnet, das des Weiteren mit einer Alarmeinrichtung 62, zum Beispiel einem Schallgeber ausgestattet ist. Der Strahlenmelder 104 wird zum Beispiel an einer Wand 4 befestigt, die einen dosimetrisch zu überwachenden Raum 5 begrenzt. Bei Überschreiten der Strahlungsdosis im Raum 5 wird vom Schallgeber 62 ein Alarmsignal abgegeben. Die Ausführungsform gemäß Figur 9 ist besonders vorteilhaft für Anwendungen als Umweltsensor für Hintergrundstrahlung oder als Sensor in Strahlungsanlagen mit Energieauflösung geeignet.

## Patentansprüche

1. Strahlungsdetektor (10), insbesondere zur Detektion von Röntgenstrahlung, umfassend:
- ein Trägersubstrat (11),
- eine Detektorschicht (12), die auf dem Trägersubstrat (11) angeordnet ist und eine Dicke geringer als 50 µm aufweist,
- Kontaktelektroden (13), die mit der Detektorschicht (12) ohmsche Kontakte bilden, und
- eine Haftoberfläche mit einer inhärenten Haftfähigkeit,
**dadurch gekennzeichnet, dass**
- die Detektorschicht (12) GaN enthält und wie ein Photoleiter wirkt, in dem in Reaktion auf Röntgenbestrahlung ein Photostrom generiert wird, der an den Kontaktelektroden (13) messbar ist, und
- der Strahlungsdetektor (10) eine RFID-Einrichtung (30) aufweist, die zur Strommessung, Datenspeicherung und Datenübertragung eingerichtet ist.

2. Strahlungsdetektor gemäß Anspruch 1, bei dem
- die Dicke der Detektorschicht (12) geringer als 10 µm, insbesondere geringer als 5 µm ist,
- die Detektorschicht (12) eine Fläche aufweist, die geringer als 100 mm², insbesondere geringer als 10 mm² ist,
- die Kontaktelektroden zwei Kontaktelektroden (13) umfassen, die einseitig auf der Detektorschicht (12) angeordnet sind, und/oder
- das GaN mit Fe oder C dotiert ist.

3. Strahlungsdetektor gemäß einem der vorhergehenden Ansprüche, der
- eine Verkapselung (20) aufweist, die insbesondere flüssigkeitsdicht, resistent gegen Säuren, resistent gegen Basen, temperaturbeständig und/oder druckbeständig ist.

4. Strahlungsdetektor gemäß Anspruch 3, bei dem
- die Verkapselung (20) eine biokompatible Oberfläche aufweist.

5. Strahlungsdetektor gemäß einem der vorhergehenden Ansprüche, bei dem
- die Haftoberfläche auf einer biegsamen Materialbahn gebildet ist, mit der das Trägersubstrat verbunden ist.

6. Strahlungsdetektor gemäß einem der vorhergehenden Ansprüche, der
- Teil einer Endoskopieeinrichtung (103) ist,
- Teil eines Klebebandes (101) ist,
- Teil einer Kultivierungseinrichtung (102) für biologische Zellen ist, und/oder
- Teil einer Implantateinrichtung ist, die zur Implantation in oder an einen lebenden Organismus konfiguriert ist.

7. Messeinrichtung (100, 101, 102, 103, 104), die mit mindestens einem Strahlungsdetektor (10) gemäß einem der vorhergehenden Ansprüche ausgestattet ist.

8. Messeinrichtung gemäß Anspruch 7, bei der
- ein Gehäuse (110) vorgesehen ist, in dem der mindestens eine Strahlungsdetektor (10) beweglich ist.

9. Messeinrichtung gemäß Anspruch 7 oder 8, die
- eine Kultivierungseinrichtung (102) oder eine Endoskopieeinrichtung (103) oder für biologische Zellen ist.

10. Messeinrichtung gemäß einem der Ansprüche 7 bis 9, bei der
- eine Vielzahl von Strahlungsdetektoren (10) vorgesehen sind, die entlang einer Bezugslinie oder einer Bezugsfläche angeordnet sind.

11. Messeinrichtung gemäß einem der Ansprüche 7 bis 10, die
- mit einer Alarmeinrichtung (62) ausgestattet ist,
- mit einer Positionierungseinrichtung (120) ausgestattet ist, die zur Positionierung des mindestens einen Strahlungsdetektors relativ zu einem zu untersuchenden Gegenstand konfiguriert ist, und/oder
- die mit einer Messwertaufnehmereinrichtung (31) ausgestattet ist, die zur wiederholten Aufnahme von Dosiswerten in Abhängigkeit von der Zeit konfiguriert ist.

12. Verwendung eines Strahlungsdetektors oder einer Messeinrichtung gemäß einem der vorhergehenden Ansprüche zur Dosimetrie von Röntgenstrahlung.

## Claims

1. Radiation detector (10), in particular for detection of X-ray radiation, comprising:
- a carrier substrate (11),
- a detector layer (12), which is arranged on the carrier substrate (11) and has a thickness of less than 50 µm,
- contact electrodes (13), which form ohmic contacts with the detector layer (12), and
- an adhesive surface with an inherent tackiness,
**characterized in that**
- the detector layer (12) contains GaN and acts like a photoconductor, in which, in response to X-ray irradiation, a photocurrent is generated, which is measurable at the contact electrodes (13), and
- the radiation detector (10) has an RFID device, which is adapted for current measurement, data storage and data transmission.

2. Radiation detector according to claim 1, wherein
- the thickness of the detector layer (12) is less than 10 µm, in particular less than 5 µm,
- the detector layer (12) has a surface, which is less than 100 mm², in particular less than 10 mm²,
- the contact electrodes comprise two contact electrodes (13), which are arranged on one side on the detector layer (12), and/or
- the GaN is doped with Fe or C.

3. Radiation detector according to any one of the preceding claims, which
- has an encapsulation (20), which is in particular liquid-tight, resistant against acids, resistant against bases, temperature-proof and/or pressure-resistant.

4. Radiation detector according to claim 3, wherein
- the encapsulation (20) has a biocompatible surface.

5. Radiation detector according to one of the preceding claims, wherein
- the adhesive surface is formed on a bendable material web, wherein the carrier substrate is connected with the material web.

6. Radiation detector according to one of the preceding claims, which
- is part of an endoscopic device (103),
- is part of an adhesive tape (101),
- is part of a cultivation device (102) for biological cells, and/or
- is part of an implant device, which is configured for implantation in or on a living organism.

7. A measuring device (100, 101, 102, 103, 104), which is provided with at least one radiation detector (10) according to one of the preceding claims.

8. Measuring device according to claim 7, wherein
- a housing (110) is provided for, in which the at least one radiation detector (10) is movable.

9. The measuring device according to claim 7 or 8, which
- is a cultivation device (102) or an endoscopic device (103) or for biological cells.

10. The measuring device according to one of claims 7 to 9, wherein
- a plurality of radiation detectors (10) are provided for, which are arranged along a reference line or a reference surface.

11. The measuring device according to any one of claims 7 to 10, which
- is provided with an alarm device (62),
- is provided with a positioning device (120), which is configured for positioning the at least one radiation detector relative to an object to be analyzed, and/or
- is provided with a transducer device (31), which is configured for repeated recording of dosage values depending on the time.

12. Use of a radiation detector or a measuring device according to one of the preceding claims for dosimetry of X-ray radiation.

## Revendications

1. Détecteur de rayonnement (10), plus particulièrement pour la détection de rayons X, comprenant :
- un substrat de support (11),
- une couche de détecteur (12), qui est disposée sur le substrat de support (11) et qui présente une épaisseur inférieure à 50 µm,
- des électrodes de contact (13) qui forment, avec la couche de détecteur (12), des contacts ohmiques et
- une surface adhésive avec une adhérence inhérente,
**caractérisé en ce que**
- la couche de détecteur (12) contient du GaN et agit comme un photoconducteur, dans lequel, en réaction à un rayonnement X, un courant photoélectrique est généré, qui peut être mesuré au niveau des électrodes de contact (13) et
- le détecteur de rayonnement (10) comprend un dispositif RFID (30) qui est conçu pour la mesure du courant, l'enregistrement des données et la transmission des données.

2. Détecteur de rayonnement selon la revendication 1, dans lequel
- l'épaisseur de la couche de détecteur (12) est inférieure à 10 m, plus particulièrement inférieure à 5 m,
- la couche de détecteur (12) présente une superficie qui est inférieure à 100 mm², plus particulièrement inférieure à 10 mm²,
- les électrodes de contact comprennent deux électrodes de contact (13) qui sont disposées sur un côté de la couche de détecteur (12) et/ou
- le GaN est dopé avec Fe ou C.

3. Détecteur de rayonnement selon l'une des revendications précédentes, qui
- comprend une encapsulation (20) qui est, plus particulièrement, étanche aux liquides, résistante aux acides, résistante aux bases, résistante à la température et/ou résistante à la pression.

4. Détecteur de rayonnement selon la revendication 3, dans lequel
- l'encapsulation (20) comprend une surface biocompatible.

5. Détecteur de rayonnement selon l'une des revendications précédentes, dans lequel
- la surface adhésive est formée sur une bande de matériau flexible, avec laquelle le substrat de support est relié.

6. Détecteur de rayonnement selon l'une des revendications précédentes, qui
- fait partie d'un dispositif d'endoscopie (103),
- fait partie d'une bande adhésive (101),
- fait partie d'un dispositif de culture (102) pour des cellules biologiques et/ou
- fait partie d'un dispositif d'implant qui est conçu pour une implantation dans ou sur un organisme vivant.

7. Dispositif de mesure (100, 101, 102, 103, 104), qui est équipé d'au moins un détecteur de rayonnement (10) selon l'une des revendications précédentes.

8. Dispositif de mesure selon la revendication 7, dans lequel
- un boîtier (110) est prévu, dans lequel l'au moins un détecteur de rayonnement (10) est mobile.

9. Dispositif de mesure selon la revendication 7 ou 8, qui
- est un dispositif de culture (102) ou un dispositif d'endoscopie (103) ou pour des cellules biologiques.

10. Dispositif de mesure selon l'une des revendications 7 à 9, dans lequel
- une pluralité de détecteurs de rayonnements (10) sont prévus, qui sont disposés le long d'une ligne de référence ou d'une surface de référence.

11. Dispositif de mesure selon l'une des revendications 7 à 10, qui
- est équipé d'un dispositif d'alarme (62),
- est équipé d'un dispositif de positionnement (120) qui est conçu pour le positionnement de l'au moins un détecteur de rayonnement par rapport à un objet à examiner et/ou
- est équipé d'un dispositif enregistreur de valeur de mesure (31) qui est conçu pour l'enregistrement répété de valeurs de doses en fonction du temps.

12. Utilisation d'un détecteur de rayonnement ou d'un dispositif de mesure selon l'une des revendications précédentes pour la dosimétrie de rayons X.
